# EUROPEAN PATENT SPECIFICATION

(11) **EP 3 577 235 B1**
(45) Date of publication and mention of the grant of the patent: **30.03.2022**
(21) Application number: 18702710.7
(22) Date of filing: 06.02.2018
(51) Int. Cl.: C12Q 1/6883

(54) **PREDICTIVE METHODS OF ASCENDING AORTA DILATION AND KITS FOR USE IN THE METHODS**
VERFAHREN ZUR VORHERSAGE AUFSTEIGENDER AORTADILATATION UND KIT ZUR VERWENDUNG IN DEN VERFAHREN
PROCÉDÉS DE PRÉDICTION DE LA DILATATION DE L'AORTE ASCENDANTE ET TROUSSE POUR L'UTILISATION DANS LES DITS PROCÉDÉS

(30) Priority: 06.02.2017 EP 17382048
(43) Date of publication of application: 11.12.2019
(73) Proprietor: Institut d'Investigació Sanitària Pere Virgili, 43003 Tarragona (ES)
(72) Inventor: ALEGRET COLOMÉ, Josep Maria, 43850 Cambrils-Tarragona (ES); MARTÍNEZ MICAELO, Neus, 43206 Reus-Tarragona (ES)
(74) Representative: ABG Intellectual Property Law, S.L.
(86) International application number: PCT/EP2018/052961
(87) International publication number: WO 2018/141990

(56) References cited:
- WO-A2-2014/004889
- US-A1- 2013 289 141
- IKEDA SADAKATSU ET AL: "Altered microRNA expression in human heart disease", PHYSIOLOGICAL GENO, AMERICAN PHYSIOLOGICAL SOCIETY, US, vol. 31, no. 3, 1 November 2007 (2007-11-01), pages 367-373, XP009098819, ISSN: 1094-8341
- IKONOMIDIS JOHN S ET AL: "Plasma biomarkers for distinguishing etiologic subtypes of thoracic aortic aneurysm disease", JOURNAL OF THORACIC AND CARDIOVASCULAR SURGERY, MOSBY-YEAR BOOK, INC., ST. LOUIS, MO, US, vol. 145, no. 5, 11 January 2013 (2013-01-11), pages 1326-1333, XP028580017, ISSN: 0022-5223, DOI: 10.1016/J.JTCVS.2012.12.027
- CHA HWA JUN ET AL: "MicroRNA expression profiling ofp-phenylenediamine treatment in human keratinocyte cell line", MOLECULAR & CELLULAR TOXICOLOGY, SEOUL : KOREAN SOCIETY OF TOXICOGENOMICS AND TOXICOPROTEOMICS, KOREA, vol. 11, no. 1, 2 April 2015 (2015-04-02), pages 19-28, XP035444282, ISSN: 1738-642X, DOI: 10.1007/S13273-015-0003-9 [retrieved on 2015-04-02]
- M. F. CORSTEN ET AL: "MicroRNA Profiling Identifies MicroRNA-155 as an Adverse Mediator of Cardiac Injury and Dysfunction During Acute Viral Myocarditis", CIRCULATION RESEARCH, vol. 111, no. 4, 3 August 2012 (2012-08-03), pages 415-425, XP055062086, ISSN: 0009-7330, DOI: 10.1161/CIRCRESAHA.112.267443
- NEUS MARTÍNEZ-MICAELO ET AL: "Specific circulating microRNA signature of bicuspid aortic valve disease", JOURNAL OF TRANSLATIONAL MEDICINE, vol. 15, no. 1, 11 April 2017 (2017-04-11) , page 76, XP055389492, DOI: 10.1186/s12967-017-1176-x

## Description

### FIELD OF THE INVENTION

The present invention is related to the technical field of diagnostics, particularly to methods for diagnosing ascending aorta dilation, determining the probability of suffering from ascending aorta dilation, predicting the progression of ascending aorta dilation and kits useful for carrying on said methods.

### BACKGROUND OF THE INVENTION

Bicuspid aortic valve (BAV) represents the most common congenital cardiac malformation and is commonly associated with both the development of aortic valve dysfunction and the progressive dilation of the ascending aorta. In patients with BAV, the dilation of the aorta is associated with aortic regurgitation and also with the risk of aortic dissection, and in consequence the often requirement of prophylactic aortic surgery. The causes of aorta dilation have been debated for several years, and changes in the flow characteristics within the ascending aorta have been suggested as the main cause. However, the mechanisms involved have not been fully elucidated.

Nowadays, in the clinical practice the diagnosis of BAV and ascending aorta dilation is performed exclusively based on image techniques (i.e. echocardiography, TAC, magnetic resonance) and the subsequent interpretation by an expert. Moreover, is also important to take in to consideration that both, the BAV and the dilation of the aorta are normally presented as asymptomatic clinical conditions and the molecular causes that are underlying both cardiopathologies are unknown.

It has been disclosed that patients who suffer from dilated cardiomyopathy can be distinguished from patients suffering from ischemic cardiomyopathy or aortic stenosis based on the expression of specific microRNAs in myocardial samples (Ikeda S. et al. Physiol Genomics 2007; 31:367-373).

Other authors have disclosed that different etiologies of thoracic aortic aneurism disease can be distinguished by determining the level of specific miRNA molecules in ascending thoracic aortic aneurysm tissue and plasma samples (Ikonomidis J.S. et al. J Thorac Cardiovasc Surg 2013; 145:1326-1333), and that the size of thoracic aortic aneurysms may be predicted by determining microRNA levels in blood or plasma (WO 2014/004889 A2).

US 2013/289141 A1 discloses the use of a universal miR microarray for determining the expression level of microRNA molecules in a tissue sample from patients who suffer from thoracic aortic aneurysms.

Previous studies have analysed the expression of miRNAs in aortic tissue segments of patients with BAV and animal models; Nigam et al. (Nigam V. et al. J Heart Valve Dis 2010; 19:459-465), determined that miR-26a, miR-30b and miR-195 were decreased in the aortic valve leaflets of patients requiring aortic valve replacement due to aortic stenosis, compared to those requiring replacement due to aortic insufficiency, both pathologies associated with BAV. Yanagawa et al. (Yanagawa B. et al. J Thorac Cardiovasc Surg 2012; 144:256-262), determined using porcine valvular interstitial cells and human valve leaflets, that miR-141 down regulation in stenotic bicuspid leaflets regulates BMP-2-mediated valve calcification. Wu et al. (Wu J. et al. J Am Coll Cardiol 2016; 67:2965-2977), performed paired comparison of the miRNA expression between severely dilated and normal appearing less dilated aortic samples, associating the differential regulation of the miR-17 gene cluster with the predisposition to dilation through the dysregulation of the TIMP-MMP pathway. In this way, although the determination of the miRNA expression profile in tissue samples provides valuable information regarding the pathophysiological mechanisms underlying diseases, in most cases this expression signature cannot be extrapolated to the bloodstream.

Thus, the development of a fast and simple screening method that can be conducted in an unbiased, high-throughput is needed for prompt diagnosis of BAV and ascending aorta dilation.

### SUMMARY OF THE INVENTION

The authors of the present invention have identified cell-free circulating miRNA miR-718 as a biomarker for ascending aorta dilation in blood samples and also for predicting the progression of a subject suffering from said disease. Outside the scope of the claimed invention, the authors have identified three circulating miRNAs (miR-130a-3p, miR-122-5p and miR-486-5p) that allow diagnosing bicuspid aortic valve in a blood sample of a subject. The serum-based diagnostic method of the invention can serve as a complement to echocardiogram for BAV screening, particularly in large populations.

Therefore, the invention relates in a first aspect to an *in vitro* method for diagnosing ascending aorta dilation in a subject that comprises
(i) determining the level of expression of circulating miR-718 in a blood sample from said subject, and
(ii) comparing the level of expression obtained in (i) with a reference value, wherein if the level of expression of circulating miR-718 is decreased with respect to the reference value, then the subject is diagnosed with ascending aorta dilation.

In a second aspect, the present invention relates to an *in vitro* method for determining the probability that a subject suffers from ascending aorta dilation that comprises
(i) determining the level of expression of circulating miR-718 in a blood sample from said subject, and
(ii) comparing the level of expression obtained in (i) with a reference value,
wherein if the level of expression of circulating miR-718 is decreased with respect to the reference value, then the subject shows high probability of suffering from ascending aorta dilation or
wherein if the level of expression of circulating miR-718 is equal to or increased with respect to the reference value, then the subject shows low probability of suffering from ascending aorta dilation.

In a third aspect, the invention relates to an *in vitro* method for determining the prognosis or for monitoring the progression of a subject suffering from ascending aorta dilation that comprises:
(i) determining the level of expression of circulating miR-718 in a blood sample from said subject, and
(ii) comparing the level of expression obtained in (i) with a reference value obtained from the same subject at an earlier time of point
wherein if the level of expression of circulating miR-718 is decreased with respect to the reference value, then the subject shows bad prognosis or
wherein if the level of expression of circulating miR-718 is increased with respect to the reference value, then the subject shows good prognosis.

In a further aspect, the invention relates to a kit comprising reagents adequate for the determination of the level of expression of miR-718, wherein the oligonucleotides that hybridize specifically to miR-718 comprise at least 50% of the total amount of oligonucleotides forming the kit.

In another aspect, the invention relates to the use of a kit for diagnosing ascending aorta dilation in a method of the invention, or for determining the probability that a subject suffers from ascending aorta dilation in a method of the invention or for determining the prognosis or for monitoring the progression of a subject suffering from ascending aorta dilation in a method of the invention, wherein the kit comprises reagents adequate for the determination of the level of expression of miR-718.

### BRIEF DESCRIPTION OF THE FIGURES

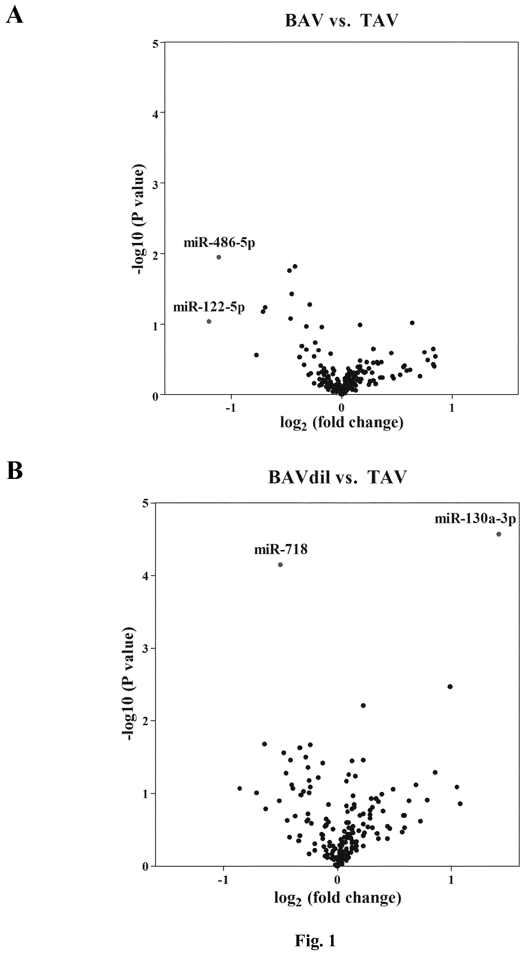

**Figure 1****.** Volcano plot representing differences in the circulating expression levels of miRNAs depending on (A) the bicuspid or tricuspid morphology of the aortic valve and (B) the morphology of the valve together with the dilation of the aorta. The log-fold change is plotted against the -log10 (P value).

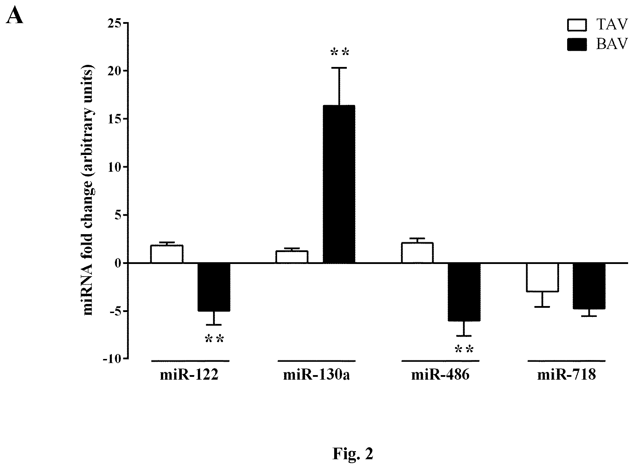

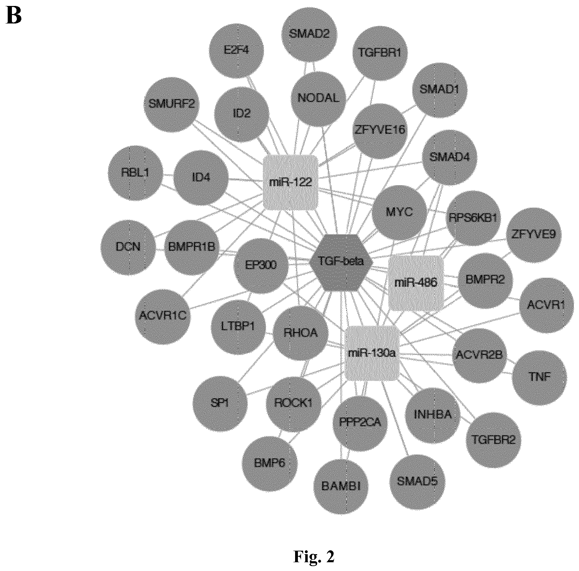

**Figure 2****.** The expression of miR-122-5p, miR-130a-3p and miR-486-5p are influenced by the morphology of the aortic valve. (A) Validation of the miRNA candidates for aortic morphology was carried out by RT-qPCR. ^{∗∗} Significant values (p<0.001; unpaired t-test). (B) Interactive miRNA-gene network regarding the TGF-beta signaling pathway. TAV; tricuspid aortic valve, BAV; bicuspid aortic valve, TGF; transforming growth factor.

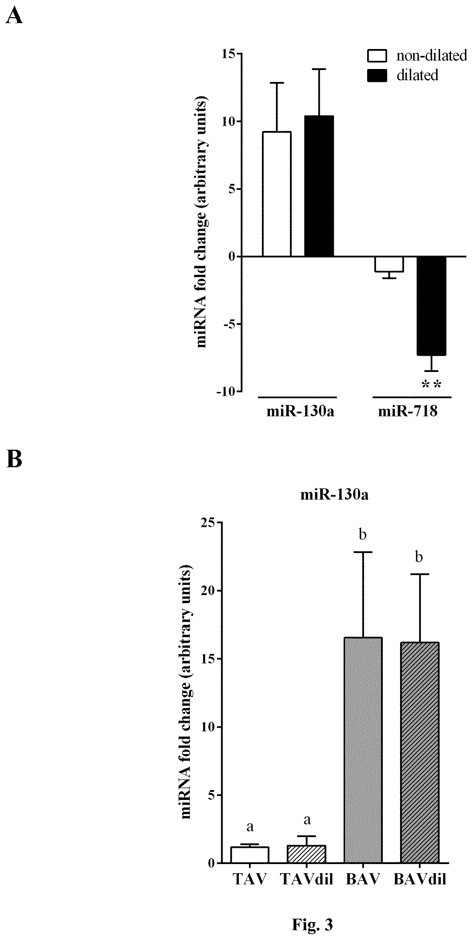

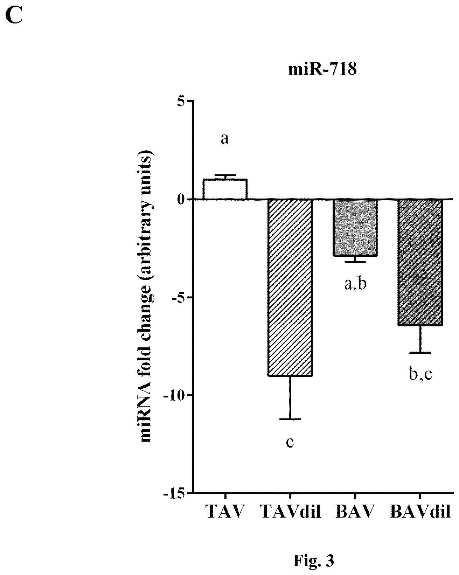

**Figure 3****.** Circulating expression of miR-718 is affected by the dilation of the ascending aorta. (A) RT-qPCR validation of the effect of the ascending aortic dilation in the expression levels of miR-130a-3p and miR-718 in plasma. Specific effects of valve morphology and aortic dilation were analyzed for miR-130a-3p (B) and miR-718 (C). ^{∗∗} Significant values (p<0.001; unpaired t-test). Bars with different letters are significantly different (one-way ANOVA, Tukey test, p<0.05).

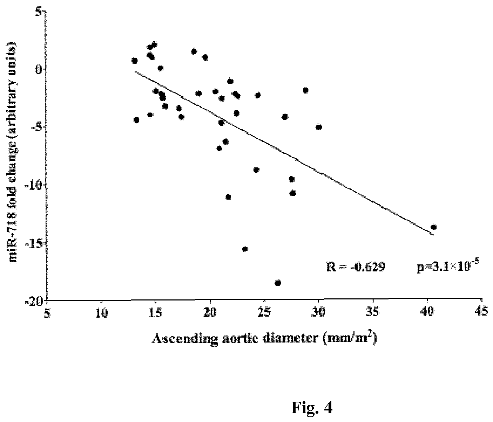

**Figure 4****.** The circulating expression of miR-718 is inversely correlated to the diameter of the ascending aorta.

### DETAILED DESCRIPTION OF THE INVENTION

### 1. Definitions

The term "ascending aorta dilation", as used herein, relates to a degenerative vascular disorder due to the destructive remodeling of the aortic wall and the degradation of the extracellular matrix proteins, leading to the recruitment and infiltration of immune system cells mediated by the secretion of adhesion molecules. The ascending aorta originates beyond the aortic valve and ends right before the innominate artery (brachiocephalic trunc). The ascending aorta dilation is a dilation of the first portion of the thoracic aorta. By common convention, aortic dilation refers to a dimension that is greater than the 95^{th} percentile for the normal person age, sex and body size. Ascending aorta dilation can be either due to an innate defect or an acquired one. Exemplary, non-limitative causes of ascending aorta dilation are shown in Table 1.

**Table 1. Etiologies of ascending aorta dilation**

| Congenital | Hereditary | Acquired |
|---|---|---|
| Bicuspid aortic valve | Connective tissue diseases | Hypertension |
| | • Marfan syndrome | |
| | • Ehlers-Danlos syndrome | Infectious |
| | • Loeys-Dietz syndrome | |
| | • Arterial tortuosity syndrome | • Syphilitic 1 |
| | • Aneurysms osteoarthritis syndrome | • Bacterial infections |
| | | • Viral |
| | | • Fungal |
| Tetralogy of Fallot | Familial aortic syndromes | Auto-immune |
| | • Autosomal dominant | • Takayasu |
| | • Sporadic | • Behcet disease |
| | | • Idiopathic aortitis |
| | | Post-traumatic |
| | | Chronic aortic dissection AV fistula Post -stenotic |

Dilatation of the ascending aorta is a very indolent process as it takes many years to develop and it is asymptomatic initially. In patients who develop an ascending aortic aneurysm secondarily to a systemic disorder, signs of the primary disease are the ones who lead the clinician to look for the dilatation such as in Marfan syndrome. Otherwise, this pathology remains quiet until its catastrophic complications occur or when it is incidentally seen on cardiovascular imaging related to other causes. We can prevent these complications by screening asymptomatic patients. Feared events include aortic dissection or rupture, pericardial hemorrhage, cardiac tamponade and occlusion of aortic branches. In addition, some patients, in a lesser proportion, can also develop intramural hematomas or penetrating aortic ulcers.

Methods to determine the dilation of the aorta are known by the skilled person and include, without limitation, imaging such as ultrasound, CT-scan, magnetic resonance imaging (MRI), chest X-ray or echocardiography.

The term "ascending aortic diameter", as used herein, relates to the diameter of the ascending aorta that can be assessed by different methods known by the person skilled in the art such as, without limitation, echocardiography, multi-detector computed tomography (MDCT) or magnetic resonance imaging (MRI). Suitable methods for imaging and measuring ascending aorta are disclosed in Goldstein S.A. et al. (Goldstein S. A. et al. Am Soc Echocardiogr 2015;28:119-82). In a preferred embodiment the ascending aortic diameter is measured by echocardiography. In another preferred embodiment the ascending aortic diameter is measured from the leading edge of the anterior root wall to the leading edge of the posterior aortic root wall at end-diastole (Goldstein S. A. et al. Am Soc Echocardiogr 2015;28:119-82). Said diameter is different depending on gender and age. The ascending aortic diameter can be the luminal diameter or the total diameter (lumen + wall) of the ascending aorta.

In a preferred embodiment the aortic size index has been used to classify subjects as having aortic dilation. The aortic size index (Davies R.R. et al. Ann Thorac Surg, 81 (2006): 169-177) takes into account the body surface area, thus minimizing classification of normal aorta as pathologically dilated and viceversa. In a preferred embodiment of the invention a subject having an aortic diameter higher than 21 mm/m² is considered to suffer from ascending aorta dilation.

The term "bicuspid aortic valve" or "BAV", as used herein, refers to a congenital cardiac anomaly. While a normal tricuspid aortic valve (TAV) consists of three functional leaflets, a BAV is formed by only two. In BAV disease two of the aortic valvular leaflets fuse during development resulting in a valve that is bicuspid, instead of the normal tricuspid configuration. In many cases, a bicuspid aortic valve will cause no problems. However, BAV is associated with valvular complications (aortic stenosis or regurgitation) as well as vascular complications such ascending aorta dilation beyond the sinotubular junction and a great percentage of patients will develop serious complications. If these become severe enough, they may require heart surgery. Methods to diagnose BAV are known by the skilled person and include, without limitation, echocardiography or magnetic resonance imaging.

The term "blood sample", as used herein, refers to biological material isolated from the blood of a subject. The blood sample can be whole blood, plasma or serum. Blood sample contains any biological material suitable for detecting RNA, particularly miRNA, and is a material comprising genetic material from the subject. The sample can comprise cell and/or non-cell material of the subject, preferably non-cell material. In the present invention, the sample comprises genetic material, e.g., DNA, genomic DNA (gDNA), complementary DNA (cDNA), RNA, heterogeneous nuclear RNA (hnRNA), mRNA, etc., from the subject under study. In the present invention, the sample comprises miRNA. Methods for obtaining blood samples are well known to those skilled in the art. In a preferred embodiment the blood sample is a plasma sample.

The term "decreased expression level", as used herein in relation to the expression level of a miRNA, particularly miR-718, miR-122-5p or miR-486-5p, relates to a situation where the level of expression of said miRNA is decreased at least 5%, at least 10%, at least 20%, at least 30%, at least 40%, at least 50%, at least 60%, at least 70%, at least 80%, at least 90% or at least 100% when compared to the corresponding reference value.

The expression "determining the probability" or "predicting", as used herein in the context of the predicting method of the invention, relates to the prediction that a subject suffers from ascending aorta dilation. As the person skilled in the art will understand, such determination does not usually seek to be correct for all (i.e., 100%) of the subjects that are going to be identified. However, the term requires that a statistically significant part of the subjects can be identified (for example, a cohort in a cohort study). The person skilled in the art can easily determine if a part is statistically significant using several well-known statistical evaluation tools, for example, determination of confidence intervals, determination of p values, Student's t-test, Mann-Whitney test, etc. The details are found in Dowdy and Wearden, Statistics for Research, John Wiley and Sons, New York 1983. The preferred confidence intervals are at least 90%, at least 95%, at least 97%, at least 98% or at least 99%. The p values are preferably 0.1, 0.05, 0.01, 0.005 or 0.0001. More preferably, at least 60%, at least 70%, at least 80% or at least 90% of the subjects of a population can be suitably identified by the method of the present invention.

The expression "determining the prognosis" or "monitoring the progression", as used herein, relates to the prediction of a medical outcome, for example, a poor or good outcome (e.g. likelihood of long-term survival, overall survival, disease-specific survival, progression-free survival or disease-free survival). A "negative prognosis", or "bad prognosis", or "poor outcome", includes a prediction of relapse, disease progression, mortality or the necessity for a surgical treatment. A "positive prognosis", or "good prognosis", or "good outcome", includes stabilization of the disease. As will be understood by those skilled in the art, the prediction, although preferred to be, need not be correct for 100% of the subjects to be evaluated. However, the term requires that a statistically significant part of the subjects can be identified as having an increased probability of having a given outcome. The person skilled in the art can easily determine if a part is statistically significant using several well-known statistical evaluation tools, for example, determination of confidence intervals, determination of p values, Student's t-test, Mann-Whitney test, etc. The details are found in Dowdy and Wearden, Statistics for Research, John Wiley and Sons, New York 1983. The preferred confidence intervals are at least 50%, at least 60%, at least 70%, at least 80%, at least 90%, at least 95%, at least 97%, at least 98% or at least 99%. The p values are preferably 0.1, 0.05, 0.01, 0.005 or 0.0001. More preferably, at least 60%, at least 70%, at least 80% or at least 90% of the subjects of a population can be suitably identified by the method of the present invention. Any parameter which is widely accepted for determining prognosis of a patient can be used in the present invention including, without limitation, overall survival time.

The term "diagnose" or "diagnosis", as used herein, relates to the evaluation of the probability according to which a subject suffers a specific pathology (in this case, suffering from ascending aorta dilation). As the skilled in the art will understand, such evaluation may not be correct for 100% of the subjects to be diagnosed, although it preferably is. The term, however, requires being able to identify a statistically significant part of the subjects.

The term "high probability", as used herein in the predicting method of the invention, relates to the situation where the subject shows at least 5 %, at least 10%, at least 20%, at least 30%, at least 40%, at least 50%, at least 60%, at least 70%, at least 80%, at least 90%, at least 100% probabilities of developing or suffering from a disease, particularly ascending aorta dilation.

The term "increased expression level", as used herein in relation to the expression level of a miRNA, particularly miR-718 or miR-130a-3p, relates to a situation where the level of expression of said miRNA is increased at least 5%, at least 10%, at least 20%, at least 30%, at least 40%, at least 50%, at least 60%, at least 70%, at least 80%, at least 90% or at least 100% when compared to the corresponding reference value.

The term "kit" or "kit-of-parts", as used herein, relates to an entity of physically separated components, which are intended for individual use, but in functional relation to each other. The kit of the invention is a product that contains the different reagents needed to carry on the different methods of the invention packed so as to allow their transport and storage. Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet addresses that provide said instructions.

The term "level of expression" relates to the measurement of the amount of a nucleic acid, e.g. RNA or mRNA, or of a protein. As used herein, the level of expression relates to the measurement of the amount of RNA, more particularly miRNA, even more particularly miR-718, miR-130a-3p, miR-122-5p or miR-486-5p.

The term "low probability", as used herein in the predicting method of the invention, relates to the situation where the subject shows at least 5 %, at least 10%, at least 20%, at least 30%, at least 40%, at least 50%, at least 60%, at least 70%, at least 80%, at least 90%, at least 100% probabilities of not developing or suffering from a disease, particularly ascending aorta dilation.

The terms "miRNA" or "microRNA", used interchangeably herein, are endogenous RNAs, some of which are known to regulate the expression of protein-coding genes at the post-transcriptional level. As used herein, the term "miRNA" refers to any type of micro-interfering RNA, including but not limited to, endogenous microRNA and artificial microRNA. Typically, endogenous miRNAs are small RNAs encoded in the genome which are capable of modulating the productive utilization of mRNA. A mature miRNA is a single-stranded RNA molecule of about 21-23 nucleotides in length which is complementary to a target sequence, and hybridizes to the target RNA sequence to inhibit its translation. miRNAs themselves are encoded by genes that are transcribed from DNA but not translated into protein (non-coding RNA); instead they are processed from primary transcripts known as pri-miRNA to short stem-loop structures called pre-miRNA and finally to functional miRNA. Mature miRNA molecules are partially complementary to one or more messenger RNA (mRNA) molecules, and their main function is to down-regulate gene expression. Besides their intracellular function, miRNAs can be exported or released by cells into the circulating blood in very stable forms. The term "circulating miRNA", as used herein, also known as "cell-free miRNA", relates to extracellular miRNA that circulates in the bloodstream.

Pre-miRNAs are referred to as "mir", whereas mature miRNAs are referred to as "miR". This prefix is followed by a dash and a number, the latter often indicating order of naming. miRNAs with nearly identical sequences except for one or two nucleotides are annotated with an additional lower case letter. Pre-miRNAs that lead to 100% identical mature miRNAs but that are located at different places in the genome are indicated with an additional dash-number suffix. Species of origin is designated with a three-letter prefix. Some "mir" can have two mature products. For example, hsa-mir-122 has two mature products, named hsa-miR-122-5p and hsa-miR-122-3p. The mature product annotated with 5p arises from the 5' arm of the mir-122 hairpin precursor, and the mature product annotated with 3p arises from the 3'arm of the mir-122 hairpin precursor. Nomenclature conventions used to describe miRNA sequences are described further in Ambros V et al., RNA, 2003, 9:277-279.

The term "miR-718", as used herein, relates to the human miRNA as defined in the mIRBase database of the University of Manchester (http://www.mirbase.org/index.shtml) (release 21 as of June 2014) under accession number MIMAT0012735, as well as to homologs thereof including, without limitation, those defined in the above mentioned database that include dog miR-718 (accession number MIMAT0009939), mouse miR-718 (accession number MIMAT0003514) and chimpanzee miR-718 (accession number MIMAT0012821).

The term "miR-130a-3p", as used herein, relates to the human miRNA as defined in the mIRBase database of the University of Manchester (http://www.mirbase.org/index.shtml) (release 21 as of June 2014) under accession number MIMAT0000425 (corresponding to mature sequence hsa-miR-130a-3p) wherein the mature sequence is excised from the 3'arm of the hairpin precursor. The term "miR-130a-3p" relates also to homologs of the human miRNA including, without limitation, those defined in the above mentioned database that include mouse miR-130a-3p (accession number MIMAT0000141) and rat miR-130a-3p (accession number MIMAT0000836).

The term "miR-122-5p", as used herein, relates to the human miRNA as defined in the mIRBase database of the University of Manchester (http://www.mirbase.org/index.shtml) (release 21 as of June 2014) under accession number MIMAT0000421 (corresponding to mature sequence hsa-miR-122-5p), wherein the mature sequence is excised from the 5'arm of the hairpin precursor. The term "miR-122-5p" relates also to homologs of the human miRNA including, without limitation, those defined in the above mentioned database that include mouse miR-122-5p (accession number MIMAT0000246) and rat miR-122-5p (accession number MIMAT0000827).

The term "miR-486-5p", as used herein, relates to the human miRNA as defined in the mIRBase database of the University of Manchester (http://www.mirbase.org/index.shtml) (release 21 as of June 2014) under accession number MIMAT0002177 (corresponding to mature sequence hsa-miR-486-5p), wherein the mature sequence is excised from the 5'arm of the hairpin precursor. The term "miR-486-5p" relates also to homologs of the human miRNA including, without limitation, those defined in the above mentioned database that include rhesus macaque miR-486-5p (accession number MIMAT0006344) and horse miR-486-5p (accession number MIMAT0013186).

The expression "non-dilated bicuspid aortic valve", as used herein, refers to a subject having bicuspid aortic valve either asymptomatic or suffering symptoms of the BAV disease, but that does not suffer from ascending aorta dilation.

The expression "non-dilated tricuspid aortic valve healthy subject", as used herein, refers to a healthy subject that has an aortic valve with a normal morphology (i.e. a tricuspid aortic valve) and that does not suffer from ascending aorta dilation.

The term "oligonucleotide", as used herein, refers to a nucleic acid having preferably at least 10 nucleotides, preferably at least 15 nucleotides, preferably at least 20 nucleotides, preferably at least 25 nucleotides and preferably no more than 100 nucleotides, including polyribonucleotides, polydeoxyribonucleotides and combinations thereof. The term "oligonucleotide" also refers to molecules formed by conventional nucleotides bound by phosphodiester conventional bonds as well as variants thereof including modifications in purines or pyrimidines or modifications in riboses or deoxyriboses designed to increase the stability of the oligonucleotide. Alternatively or additionally, the oligonucleotides may contain modified bonds such as phosphotriester, phosphorothioate, methylphosphonate, etc, or may be peptide nucleic acids (PNA). Methods to synthesize oligonucleotides are well known by the person skilled in the art.

The term "pharmacological treatment", as used herein, refers to a treatment comprising the administration of a drug to a subject to prevent, relieve and/or cure a disease, or to relieve, reduce or eliminate one or more symptoms associated with said disease, or to relieve, reduce or eliminate a risk factor for the progression of said disease, for example by treating a concomitant disease.

The expression "reagent adequate for the determination of the level of expression of a miRNA", as used herein, refers to any compound or set of compounds that allows the specific determination of the expression level of a miRNA by detection methods well known by the person skilled in the art.

The expression "reagent adequate for the determination of the level of expression a reference RNA", as used herein, refers to any compound or set of compounds that allows the specific determination of the expression level of a reference RNA by detection methods well known by the person skilled in the art.

The term "reference value" or "reference level", as used herein in the context of the methods of the invention, relates to a value used as a reference for the expression level values/data obtained from blood samples of subjects to be diagnosed with a disease or to be classified as having high or low probability of suffering from a disease, or to having good or bad prognosis. The reference value or reference level according to the any of the methods of the invention can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from a population of subjects of the chronological age matched group, or based on a pool of samples including or excluding the sample to be tested. Various considerations are taken into account when determining the reference value of the marker. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

The term "subject" or "individual" or "animal" or "patient" or "mammal" is meant any subject, particularly a mammalian subject, for whom diagnosis or prognosis is desired. Mammalian subjects include humans, domestic animals, farm animals, and zoo, sports, or pet animals such as dogs, cats, guinea pigs, rabbits, rats, mice, horses, cattle, cows, and so on. In a preferred embodiment of the invention, the subject is a mammal. In a more preferred embodiment of the invention, the subject is a human.

The term "tricuspid aortic valve", as used herein, refers to the normal morphology of the aortic valve, which is a valve in the human heart between the left ventricle and the aorta that normally has three cusps or leaflets.

### 2. Method for diagnosing ascending aorta dilation

The authors of the present invention have found that circulating miRNA miR-718 is a biomarker for ascending aorta dilation identification in patients.

Thus, in a first aspect, the invention relates to an *in vitro* method for diagnosing ascending aorta dilation in a subject (first method of the invention), said method comprising
(i) determining the level of expression of circulating miR-718 in a blood sample from said subject, and
(ii) comparing the level of expression obtained in (i) with a reference value, wherein if the level of expression of circulating miR-718 is decreased with respect to the reference value, then the subject is diagnosed with ascending aorta dilation.

In a first step of the method of the invention for diagnosing ascending aorta dilation, the expression level of circulating miR-718 is determined in a blood sample from a subject whose diagnosis is to be determined.

Before analyzing the sample, it will often be desirable to perform one or more preparation operations upon said sample aimed at separating the molecule to be determined (RNA, in particular miRNA) from other molecules found in the sample. Typically, these sample preparation operations include manipulations such as concentration, suspension, extraction of intracellular material, e.g., nucleic acids from tissue/whole cell samples and the like, amplification of nucleic acids, fragmentation, transcription, labeling and/or extension reactions. Nucleic acids, especially RNA can be isolated using any techniques known in the art. There are two main methods for isolating RNA: (i) phenol-based extraction and (ii) silica matrix or glass fiber filter (GFF)-based binding. Phenol-based reagents contain a combination of denaturants and RNase inhibitors for cell and tissue disruption and subsequent separation of RNA from contaminants. Phenol-based isolation procedures can recover RNA species in the 10-200-nucleotide range e.g., miRNAs, 5S rRNA, 5.8S rRNA, and U1 snRNA. If a sample of "total" RNA was purified by the popular silica matrix column or GFF procedure, it may be depleted in small RNAs. Extraction procedures such as those using Trizol or TriReagent, however will purify all RNAs, large and small, and are the recommended methods for isolating total RNA from biological samples that will contain miRNAs/siRNAs. These methods are typically well known by a person skilled in the art. There are also commercial kits available for miRNA purification including, without limitation, miRNeasy Mini kit from Qiagen, miRNA isolation kits from Life Technologies, mirPremier microRNA isolation kit from Sigma-Aldrich and High Pure miRNA isolation kit from Roche.

The level of expression of miR-718 is determined in a blood sample from a subject, wherein said sample is any blood sample comprising nucleic acids, particularly comprising circulating miRNA. In a particular embodiment, the blood sample is a cell-free sample. In a preferred embodiment the blood sample is a plasma sample.

Determination of the levels of RNA, in particular the levels of miRNA, can be carried out by any method known in the art such as qPCR, northern blot, RNA dot blot, TaqMan, tag based methods such as serial analysis of gene expression (SAGE) including variants such as LongSAGE and SuperSAGE, microarrays. Determination of the mRNA levels can also be carried out by fluorescence in situ hybridization (FISH). The detection can be carried out in individual samples or in tissue microarrays. In a particular embodiment, the expression levels of miR-718 in a blood sample from a subject to be diagnosed with ascending aorta dilation are determined by real-time PCR (RT-PCR). In another particular embodiment, the expression levels of miR-718 are determined by miRNA microarray.

In order to normalize the values of expression of miRNA among the different samples, it is possible to compare the expression levels of the miRNA of interest in the test samples with the expression of a control RNA or reference RNA. Thus, in the context of the invention, the term "normalized" expression level refers to the expression level of a miRNA, particularly miR-718, relative to the expression level of a single reference or control RNA, or a particular set of reference or control RNAs.

A "control RNA" or a "reference RNA", as used herein, relates to RNA whose expression level does not change or changes only in limited amounts in samples under analysis with respect to control samples. In the analysis of circulating miRNAs, various strategies can be used for data normalization, ranging from the use of particular endogenous miRNA or small RNA controls, external spike-in molecules or measures of central tendency. When quantifying *cellular* miRNAs, stable small RNA controls are currently used as reference RNAs. These include small noncoding RNAs, and specifically small nuclear RNA and small nucleolar RNA such as SNORD44 (RNU44), SNORD48 (RNU48) and RNU6-1 (Mamm U6). For *serum* miRNAs, there is growing evidence that the above-mentioned small RNAs are highly variable or not stably detectable, thus leading to the search for suitable stable control miRNAs that are firmly detectable in human serum. The most common choice could be the use of a miRNA that does not vary considerably between individuals. In a preferred embodiment, a synthetic miRNA is spiked into the RNA prep to help monitor RNA recovery and reverse transcription efficiency. Examples of spike-in controls are C. *elegans* cel-miR-39 or cel-miR-54 miRNA mimics that can be easily detected by real-time RT-PCR. In one embodiment, relative miRNA expression quantification is calculated according to the comparative threshold cycle (Ct) method using cel-miR-54 or cel-miR-39 as spike-in controls. Final results are determined according to the formula 2-ΔCt, where ΔCT values of the sample are determined by subtracting the Ct value of the target gene from the value of the control miRNA. In a preferred embodiment the reference RNA is the U6 small nuclear RNA (U6 snRNA).

In a second step of the method of the invention for diagnosing ascending aorta dilation, the level of expression of circulating miR-718 in a blood sample from a subject whose diagnosis is to be determined is compared to a reference value, wherein if the level of expression of circulating miR-718 is decreased with respect to the reference value, then the subject is diagnosed with ascending aorta dilation.

In a particular embodiment of the first diagnostic method of the invention, the reference value is the expression level of the miR-718 determined in a blood sample from a control subject, wherein the control subject is a healthy subject or a subject who has not been diagnosed with ascending aorta dilation. In another embodiment, the reference value is the expression level of the miR-718 as an average value determined in a pool of healthy subjects or in a pool of subjects who have not been diagnosed with ascending aorta dilation. Said reference value sample is typically obtained by combining equal amounts of samples from a subject population. In a particular preferred embodiment, the reference value is the level of expression of miR-718 determined in a blood sample from one or more non-dilated tricuspid aortic valve healthy subjects. Ascending aorta dilation is a common vascular complication of patients suffering from bicuspid aortic valve. Therefore, in another particular preferred embodiment, the reference value is the level of expression of miR-718 determined in a blood sample from one or more subjects suffering from a non-dilated bicuspid aortic valve. In another embodiment, the reference value is the level of expression of miR-718 determined in a blood sample from one or more subjects having an aortic diameter lower than 19 mm/m². Methods to determine the dilation of the ascending aorta have been disclosed in the definitions section.

Once the reference value of miR-718 expression is established, the expression levels of circulating miR-718 expressed in a blood sample from a subject to be diagnosed can be compared with this reference value, and thus be assigned as "increased", "decreased" or "equal". For example, an increase in expression levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level. On the other hand, a decrease in expression levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" expression level. Expression levels can be seen as "equal" to the reference value if the levels differ with respect to the reference value less than 5%, less than 4%, less than 3%, less than 2%, less than 1%, less than 0.5 %, less than 0.4%, less than 0.3%, less than 0.1%, less than 0.05%, or less.

Thus, according to the first diagnostic method of the invention, if there is a decrease in the expression level of circulating miR-718 in a blood sample of a subject whose diagnosis is to be determined with respect to the reference value, then said subject is diagnosed with ascending aorta dilation.

The person skilled in the art will understand that the diagnosis may not be correct for 100% of patients under study. However, the expression requires that the diagnosis method provides correct results for a statistically significant portion of patients. Determination whether the method of the invention provides statistically significant predictions can be carried out by using standard statistical techniques such as the determination of confidence intervals, determination of p value, Student's t-test, Mann-Whitney test. Suitable confidence intervals are at least 50%, at least 60%, at least 70%, at least 80%, at least 90%, at least 95%. p values are, preferably, 0.05, 0.01, 0.005.

Although ascending aorta dilation occurs more frequently and at a younger age in patients with bicuspid aortic valves (BAV) than it does in patients with normal trileaflet aortic valves (TAV), TAV subjects can also suffer from ascending aorta dilation since there are other causes of this disease.

Therefore, in an embodiment the subject diagnosed with ascending aorta dilation is a subject having a bicuspid aortic valve. In another embodiment the subject diagnosed with ascending aorta dilation is a subject having a tricuspid aortic valve. In another embodiment the subject diagnosed with ascending aorta dilation has an aortic diameter higher than 21 mm/m².

### 3. Method for determining the probability of ascending aorta dilation

The inventors have found that the levels of circulating miR-718 in a blood sample can also be used as a marker for determining the risk of suffering ascending aorta dilation.

Thus, in another aspect, the invention relates to an *in vitro* method for determining the probability that a subject suffers from ascending aorta dilation (second method of the invention, or ascending aorta dilation probability prediction method of the invention), said method comprising
(i) determining the level of expression of circulating miR-718 in a blood sample from said subject, and
(ii) comparing the level of expression obtained in (i) with a reference value, wherein if the level of expression of circulating miR-718 is decreased with respect to the reference value, then the subject shows high probability of suffering from ascending aorta dilation or
wherein if the level of expression of circulating miR-718 is equal to or increased with respect to the reference value, then the subject shows low probability of suffering from ascending aorta dilation.

In a first step of the second method of the invention, the expression level of circulating miRNA miR-718 is determined in a blood sample from a subject whose probability of suffering from ascending aorta dilation is to be determined.

Methods to determine the level of expression of a miRNA, particularly miR-718, have been described previously in the context of the first diagnostic method of the invention and are also applicable to the second method of the invention. In a particular embodiment, miRNA expression levels, particularly miR-718 levels, are determined by RT-PCR. In another embodiment, miRNA expression levels are determined by miRNA microarray.

Suitable samples to determine miRNA expression levels, particularly miR-718 levels, have been described previously in the context of the first diagnostic method of the invention and also applicable to the second method of the invention. In a particular embodiment, the blood sample is a plasma sample.

In a second step of the second method of the invention, the level of expression of circulating miR-718 in a blood sample from a subject whose ascending aorta dilation probability is to be determined is compared to a reference value, wherein if the level of expression of circulating miR-718 is decreased with respect to the reference value, then the subject shows high probability of suffering from ascending aorta dilation, and wherein if the level of expression of circulating miR-718 is equal to or increased with respect to the reference value, then the subject shows low probability of suffering from ascending aorta dilation. In a particular embodiment of the ascending aorta dilation probability prediction method of the invention, the reference value is the expression level of the miR-718 determined in a sample from a control subject, wherein the control subject is a healthy subject or a subject who has not suffered from ascending aorta dilation. In another embodiment, the reference value is the expression level of the miR-718 as an average value determined in a pool of healthy subjects or in a pool of subjects who have not suffered from ascending aorta dilation. Said reference value sample is typically obtained by combining equal amounts of samples from a subject population. In a particular preferred embodiment, the reference value is the level of expression of miR-718 determined in a blood sample from one or more non-dilated tricuspid aortic valve healthy subjects. In another particular preferred embodiment, the reference value is the level of expression of miR-718 determined in a blood sample from one or more subjects suffering from a non-dilated bicuspid aortic valve. In another preferred embodiment the reference value is the level of expression of miR-718 determined in a blood sample from one or more subjects having an aortic diameter lower than 19 mm/m². Methods to determine the dilation of the ascending aorta have been disclosed in the definitions section.

Once the reference value of miR-718 expression is established, the expression levels of circulating miR-718 expressed in a blood sample from a subject whose ascending aorta dilation probability is to be determined can be compared with this reference value, and thus be assigned a level of "increased" or "decreased", as indicated above in the context of the first method of the invention.

Thus, according to the second method of the invention, if there is a decrease in the expression level of circulating miR-718 in a blood sample of a subject whose ascending aorta dilation probability is to be determined with respect to the reference value, then said subject shows a high probability of suffering from ascending aorta dilation. If there is no variation or an increase in the expression level of circulating miR-718 in a blood sample of a subject whose ascending aorta dilation probability is to be determined with respect to the reference value, then said subject shows a low probability of suffering from ascending aorta dilation.

A high probability of developing ascending aorta dilation is understood to mean the situation where the subject shows at least 5 %, at least 10%, at least 20%, at least 30%, at least 40%, at least 50%, at least 60%, at least 70%, at least 80%, at least 90%, at least 100% probabilities of suffering from ascending aorta dilation over time.

A low probability of developing ascending aorta dilation is understood to mean the situation where the subject shows at least 5 %, at least 10%, at least 20%, at least 30%, at least 40%, at least 50%, at least 60%, at least 70%, at least 80%, at least 90%, at least 100% probabilities of not suffering from ascending aorta dilation over time.

The person skilled in the art will understand that the prediction may not be correct for 100% of patients under study. However, the expression requires that the prediction method provides correct results for a statistically significant portion of patients. Determination whether the method of the invention provides statistically significant predictions can be carried out by using standard statistical techniques such as the determination of confidence intervals, determination of p value, Student's t-test, Mann-Whitney test. Suitable confidence intervals are at least 50%, at least 60%, at least 70%, at least 80%, at least 90%, at least 95%. p values are, preferably, 0.05, 0.01, 0.005.

Embodiments disclosed in the context of the first method of the invention are also applicable to the second method of the invention.

### 4. Method for predicting the progression of ascending aorta dilation

Ascending aortic dilation has a propensity for dissection and rupture, making it a potentially lethal disease. Therefore, ascending aortic dilation warrants frequent monitoring, with possible early prophylactic surgical intervention.

The authors of the present invention have shown that the levels of miR-718 are not only useful for the diagnosis of ascending aorta dilation, but also that this marker can be monitored after the subject has been diagnosed of this disease and that the change with time of the levels of the marker predict whether the ascending aorta dilation is progressing in the subject or not. Particularly, the inventors found that the plasma expression of miR-718 is a biomarker of aortic dilation, being associated increased diameters of ascending aorta with the down-regulation of miR-718.

Thus, in another aspect, the invention relates to an *in vitro* method for determining the prognosis or for monitoring the progression of a subject suffering from ascending aorta dilation (ascending aorta dilation disease progression predictive method of the invention, or third method of the invention), wherein the method comprises
(i) determining the level of expression of circulating miR-718 in a blood sample from said subject, and
(ii) comparing the level of expression obtained in (i) with a reference value obtained from the same subject at an earlier time of point
wherein if the level of expression of circulating miR-718 is decreased with respect to the reference value, then the subject shows bad prognosis or
wherein if the level of expression of circulating miR-718 is increased with respect to the reference value, then the subject shows good prognosis.

In a particular embodiment, the subject for whom the progression of the ascending aorta dilation is to be predicted is following a pharmacological treatment. Pharmacological treatment for mild to moderate ascending aorta dilation includes beta blockers, ACE inhibitors or treatments for preventing and treating risk factors associated to ascending aorta dilation such as hypertension or dyslipidemia.

In another particular embodiment, the subject for whom the progression of the ascending aorta dilation is to be predicted has been submitted to a surgical treatment, preferably to a prophylactic aortic surgery. Surgical treatment includes, without limitation, tube graft replacement, the Bentall procedure (composite valve graft replacement with reimplantation of the coronary arteries) or the valve sparing procedure (aortic valve reimplantation or aortic valve remodeling). Such surgical procedures are well known by those skilled in the art.

In a first step of the third method of the invention, the expression level of circulating miRNA miR-718 is determined in a blood sample from a subject for whom progression of ascending aorta dilation is to be predicted.

Methods to determine the level of expression of a miRNA, particularly miR-718, have been described previously in the context of the diagnostic method of the invention and are applicable to the third method of the invention. In a particular embodiment, miRNA expression levels, particularly miR-718 levels, are determined by RT-PCR. In another embodiment, miRNA expression levels are determined by miRNA microarray.

Suitable samples to determine miRNA expression levels, particularly miR-718 levels, have been described previously in the context of the diagnostic method of the invention and are applicable to the third method of the invention. In a particular embodiment, the sample is a plasma sample.

In a second step of the third method of the invention, the level of expression of circulating miR-718 in a blood sample from a subject for whom progression of ascending aorta dilation is to be predicted is compared to a reference value obtained from a sample obtained from the same subject at an earlier time of point, wherein if the expression level of circulating miR-718 is decreased with respect to the reference value, then the subject shows bad prognosis. Alternatively, if the expression level of circulating miR-718 is increased with respect to the reference value, then the subject shows good prognosis.

In a particular embodiment of the method according to the invention to predict progression of ascending aorta dilation, the reference value is the expression level of the miR-718 determined in a blood sample from the subject for whom progression of ascending aorta dilation is to be predicted before a surgical procedure and/or before administration of a pharmacological treatment.

Once the reference value of miR-718 expression is established, the expression levels of circulating miR-718 expressed in a blood sample from a subject whose ascending aorta dilation probability is to be determined can be compared with this reference value, and thus be assigned a level of "increased" or "decreased", as indicated above in the context of the first method of the invention.

Thus, according to the third method of the invention, if there is an increase in the expression level of circulating miR-718 in a blood sample of a subject for whom progression of ascending aorta dilation is to be predicted with respect to the reference value, then the subject shows good prognosis. If there is a decrease in the expression level of circulating miR-718 in a blood sample of a subject for whom progression of ascending aorta dilation is to be predicted with respect to the reference value, then the subject shows bad prognosis.

A good prognosis of a subject suffering from ascending aorta dilation is understood as stabilization or remission of the disease, particularly because the dilation of the aorta has decreased. Particularly, a good prognosis is understood as no increase of the diameter of the aorta or a decrease of the diameter of the aorta compared to a previous measure, preferably at 6 and 12 months after diagnosis.

A bad prognosis of a subject suffering from ascending aorta dilation is understood as prediction of relapse, disease progression, mortality or the necessity for a surgical treatment, particularly because the dilation of the aorta has increased. Particularly, a bad prognosis is understood as an increase of the diameter of the aorta compared to a previous measure, preferably at 6 and 12 months after diagnosis. A bad prognosis also is understood as dissection or rupture of ascending aorta.

The person skilled in the art will understand that the prediction may not be correct for 100% of patients under study. However, the expression requires that the prediction method provides correct results for a statistically significant portion of patients. Determination whether the method of the invention provides statistically significant predictions can be carried out by using standard statistical techniques such as the determination of confidence intervals, determination of p value, Student's t-test, Mann-Whitney test. Suitable confidence intervals are at least 50%, at least 60%, at least 70%, at least 80%, at least 90%, at least 95%. p values are, preferably, 0.05, 0.01, 0.005.

Embodiments disclosed in the context of the first and second methods of the invention are also applicable to the third method of the invention.

### 5. Method for diagnosing bicuspid aortic valve not being part of the present invention

The authors have found that circulating miRNA miR-130a-3p, miR-122-5p and miR-486-5p are independent biomarkers for bicuspid aortic valve identification in patients.

Thus, it is disclosed an *in vitro* method for diagnosing bicuspid aortic valve in a subject (not being part of the present invention), said method comprising
(i) determining the level of expression of at least one circulating miRNA selected from the group consisting of miR-130a-3p, miR-122-5p and miR-486-5p in a blood sample from said subject, and
(ii) comparing the level of expression of said at least one circulating miRNA with a reference value,
wherein if the level of expression of at least one circulating miRNA selected from miR-122-5p and miR-486-5p is decreased with respect to the reference value or the level of expression of miR-130a-3p is increased with respect to the reference value, then the subject is diagnosed with bicuspid aortic valve.

In a first step of the method for diagnosing bicuspid aortic valve not being part of the present invention, the expression level of at least one circulating miRNA selected from the group consisting of miR-130a-3p, miR-122-5p and miR-486-5p is determined in a blood sample from a subject whose diagnosis is to be determined.

The expression "at least one circulating miRNA selected from the group consisting of miR-130a-3p, miR-122-5p and miR-486-5p", as used herein, means that according to the present method not being part of the present invention the level of expression of one, two or three of said miRNAs can be determined. In a particular embodiment, the method not being part of the present invention comprises the determination of the levels of expression of one of said markers. In an embodiment of the method not being part of the present invention, the marker determined is miR-130a-3p. In another embodiment of the method not being part of the present invention, the marker determined is miR-122-5p. In another embodiment of the method not being part of the present invention, the marker determined is miR-486-5p. In another particular embodiment, the method not being part of the present invention comprises determining the level of expression of two of said miRNA, particularly the determination of the levels of miR-130a-3p and miR-122-5p, or the levels of miR-130a-3p and miR-486-5p, or the levels of miR-122-5p and miR-486-5p. In another particular embodiment, the method not being part of the present invention comprises determining the levels of expression of the three miRNA, i.e. the levels of expression of miR-130a-3p, miR-122-5p and miR-486-5p. In another particular embodiment the method not being part of the present invention comprises determining the level of expression of further miRNAs different from miR-130a-3p, miR-122-5p and miR-486-5p.

Methods to determine the level of expression of a miRNA, particularly miR-130a-3p, miR-122-5p or miR-486-5p, have been described previously in the context of the first diagnostic method of the invention and are applicable to themethod not being part of the present invention. In a particular embodiment of the method not being part of the present invention, miRNA expression levels, particularly miR-130a-3p, miR-122-5p or miR-486-5p levels, are determined by RT-PCR. In another embodiment of the method not being part of the present invention, miRNA expression levels are determined by miRNA microarray.

Suitable samples to determine miRNA expression levels, particularly miR-130a-3p, miR-122-5p or miR-486-5p levels, have been described previously in the context of the first diagnostic method of the invention and are applicable to the method not being part of the present invention. In a particular embodiment of the method not being part of the present invention, the sample is a plasma sample.

Suitable methods to normalize the values of expression of miRNA among the different samples have been described previously in the context of the first diagnostic method of the invention and are applicable to the method not being part of the present invention.

In a second step of the method for diagnosing bicuspid aortic valve not being part of the present invention, the level of expression of at least one circulating miRNA selected from the group consisting of miR-130a-3p, miR-122-5p and miR-486-5p in a blood sample from a subject whose diagnosis is to be determined is compared to a reference value, wherein if the level of expression of at least one circulating miRNA selected from miR-122-5p and miR-486-5p is decreased with respect to the reference value or the level of expression of miR-130a-3p is increased with respect to the reference value, then the subject is diagnosed with bicuspid aortic valve.

In a particular embodiment of the diagnostic method not being part of the present invention, the reference value is the expression level of miR-130a-3p, miR-122-5p or miR-486-5p determined in a blood sample from a control subject, wherein the control subject is a healthy subject or a subject who has not been diagnosed with bicuspid aortic valve. In another embodiment of the method not being part of the present invention, the reference value is the expression level of the miR-130a-3p, miR-122-5p or miR-486-5p as an average value determined in a pool of healthy subjects or in a pool of subjects who have not been diagnosed with bicuspid aortic valve. Said reference value sample is typically obtained by combining equal amounts of samples from a subject population. In a preferred embodiment of the method not being part of the present invention, the reference value is the level of expression of miR-130a-3p, miR-122-5p or miR-486-5p determined in a blood sample from one or more tricuspid aortic valve healthy subjects, preferably from non-dilated tricuspid aortic valve healthy subj ects.

Once the reference value of miR-130a-3p, miR-122-5p or miR-486-5p expression is established, the expression levels of circulating miR-130a-3p, miR-122-5p or miR-486-5p expressed in a blood sample from a subject to be diagnosed can be compared with this reference value, and thus be assigned as "increased", "decreased" or "equal". For example, an increase in expression levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression level. On the other hand, a decrease in expression levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" expression level. Expression levels can be seen as "equal" to the reference value if the levels differ with respect to the reference value less than 5%, less than 4%, less than 3%, less than 2%, less than 1%, less than 0.5 %, less than 0.4%, less than 0.3%, less than 0.1%, less than 0.05%, or less.

Thus, according to the method not being part of the present invention, if there is a decrease in the expression level of circulating miR-122-5p or miR-486-5p in a blood sample of a subject whose diagnosis is to be determined with respect to the reference value, then said subject is diagnosed with bicuspid aortic valve.

Thus, according to the method not being part of the present invention, if there is an increase in the expression level of circulating miR-130a-3p in a blood sample of a subject whose diagnosis is to be determined with respect to the reference value, then said subject is diagnosed with bicuspid aortic valve.

The person skilled in the art will understand that the diagnosis may not be correct for 100% of patients under study. However, the expression requires that the diagnosis method provides correct results for a statistically significant portion of patients. Determination whether the method provides statistically significant predictions can be carried out by using standard statistical techniques such as the determination of confidence intervals, determination of p value, Student's t-test, Mann-Whitney test. Suitable confidence intervals are at least 50%, at least 60%, at least 70%, at least 80%, at least 90%, at least 95%. p values are, preferably, 0.05, 0.01, 0.005.

Embodiments disclosed in the context of the first, second and third methods of the invention are also applicable to themethod not being part of the present invention.

### 6. Kits of the invention and uses thereof

The invention also relates to kits for carrying on the methods of the invention. Therefore, in a fifth aspect, the invention relates to a kit comprising reagents adequate for the determination of the level of expression of miR-718, wherein the oligonucleotides that hybridize specifically to miR-718 comprise at least 50% of the total amount of oligonucleotides forming the kit.

In further embodiments, the reagents adequate for the determination of the expression levels of said miRNA comprise at least 55% at least 60%, at least 65%, at least 70%, at least 75%, at least 80%, at least 90%, at least 95%, at least 96%, at least 97%, at least 98% or at least 99%of the total amount of oligonucleotides forming the kit.

In another embodiment, the kit comprises reagents adequate for the determination of the level of expression of miR-718 and miR-130a-3p. In another embodiment, the kit comprises reagents adequate for the determination of the level of expression of miR-718 and miR-122-5p. In another embodiment, the kit comprises reagents adequate for the determination of the level of expression of miR-718 and miR-486-5p. In another embodiment, the kit comprises reagents adequate for the determination of the level of expression of miR-718, miR-130a-3p and a further miRNA selected from miR-122-5p and miR-486-5p. In another embodiment, the kit comprises reagents adequate for the determination of the level of expression of miR-718, miR-122-5p and a further miRNA selected from miR-130a-3p and miR-486-5p. In another embodiment, the kit comprises reagents adequate for the determination of the level of expression of miR-718, miR-486-5p and a further miRNA selected from miR-130a-3p and miR-122-5p. In another embodiment the kit comprises reagents adequate for the determination of the level of expression of miR-718, miR-130a-3p, miR-122-5p and miR-486-5p.

In a preferred embodiment, the reagents adequate for the determination of the level of expression of an miRNA are oligonucleotides that hybridize specifically to said miRNA.

In a preferred embodiment, the oligonucleotides that hybridize specifically to miR-718 comprise at least 55%, at least 60%, at least 65%, at least 70%, at least 75%, at least 80%, at least 90%, at least 95%, at least 96%, at least 97%, at least 98% or at least 99% of the total amount of oligonucleotides forming the kit.

In a preferred embodiment, the oligonucleotides that hybridize specifically to miR-130a-3p comprise at least 1%, at least 5%, at least 10%, at least 15%, at least 20%, at least 25%, at least 30%, at least 35%, at least 40%, or at least 45%, of the total amount of oligonucleotides forming the kit.

In a preferred embodiment, the oligonucleotides that hybridize specifically to miR-122-5p comprise at least 1%, at least 5%, at least 10%, at least 15%, at least 20%, at least 25%, at least 30%, at least 35%, at least 40%, or at least 45% of the total amount of oligonucleotides forming the kit.

In a preferred embodiment, the oligonucleotides that hybridize specifically to miR-486-5p comprise at least 1%, at least 5%, at least 10%, at least 15%, at least 20%, at least 25%, at least 30%, at least 35%, at least 40%, or at least 45% of the total amount of oligonucleotides forming the kit.

In a preferred embodiment, the oligonucleotides that hybridize specifically to miR-718, miR-130a-3p, miR-122-5p and miR-486-5p comprise together at least 50%, at least 55%, at least 60%, at least 65%, at least 70%, at least 75%, at least 80%, at least 90%, at least 95%, at least 96%, at least 97%, at least 98% or at least 99% of the total amount of oligonucleotides forming the kit.

These oligonucleotides are sequence-specific nucleic acids, such that they only quantify the sequence hybridizing with them and not all the double-stranded DNA. The oligonucleotide can be a primer or a probe.

Probes must be used for determining the expression level by means of PCR. A probe is an oligonucleotide having a defined sequence capable of specifically hybridizing with a complementary sequence of a nucleic acid, so it can be used for detecting and identifying complementary or substantially complementary sequences in nucleic acids. The length of the probe of the invention can vary within a large range although for practical reasons, probes having a small length of less than 10 nucleotides are preferred, preferably at least 15 nucleotides, preferably at least 20 nucleotides, preferably at least 25 nucleotides and, preferably not more than 100 nucleotides, more preferably comprised between 15 bases and 30 bases, preferably between 16 bases and 22 bases.

Alternatively or additionally, the oligonucleotides used can contain modified bonds such as phosphodiester, phosphotriester, phosphorothioate, phosphorodithioate, phosphoroselenoate, phosphorodiselenoate, phosphoroanilothioate, phosphoroamidate, methylphosphonate, boranophosphonate bonds, as well as combinations thereof, or they are peptide nucleic acids (PNAs), in which the different nucleotides are bound by amide bonds.

In case that the oligonucleotide is a probe, said probe can have at its 3' end a fluorophore and at its 5' end a molecule blocking its fluorescence emission (quencher). Said probe hybridizes specifically in the central part of the PCR product to be obtained.

The kit can contain a set of one or more pairs of oligonucleotide primers designed to specifically amplify the miRNAs of the methods of the invention.

The present invention further provides a kit containing a microarray that has miRNA-specific probe oligonucleotides.

The microarrays contained in the kit may also include probe oligonucleotides which specifically recognize RNAs suitable for normalization purposes. In certain embodiments the kit further contains a control sample. This sample can be derived from a control or normal cell, tissue, or subject, e.g., one that exhibits, for example, normal traits. In additional embodiments, the kits of the invention contain instructions for use. In an alternative embodiment, the present invention provides a kit containing a set of one or more pairs of oligonucleotide primers designed to specifically amplify the miRNAs of an miRNA signature identified using the methods of the invention in a reverse- transcriptase polymerase chain reaction assay, preferably a real-time quantitative reverse-transcriptase polymerase chain reaction assay.

The expression "hybridize specifically" refers to such conditions that allow the hybridization of two polynucleotides of single chain, of complementary sequence or having a high grade of similarity, under stringent conditions or moderately stringent conditions, producing a double chain single molecule by base pairing.

"Stringency" of hybridization reactions are readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995). Stringent conditions and moderately stringent conditions are identified as described by Sambrook et al., Molecular Cloning: A Laboratory Manual, New York: Cold Spring Harbor Press, 1989.

In another embodiment, the kit further comprises reagents adequate for the determination of the level of expression of one or more reference RNAs and/or reagents adequate for the determination of the level of expression of one or more constitutive genes.

The term "reference RNA" has been defined in the context of the present invention. The term "constitutive gene", as used herein, refers to a housekeeping gene encoding a constitutively expressed protein and carrying out essential cell functions. Housekeeping genes suitable for use as internal standards include, but are not limited to, myosin, β-2-microglobulin, ubiquitin, 18S ribosomal protein, cyclophyllin, glyceraldehyde-3-phosphate dehydrogenase (GAPDH) and actin.

The kits of the invention also contain other reagents that allow determining the expression level of a miRNA but that are not specific for said miRNA, e.g. reagents for the extraction of RNA material, etc., e.g., primers for the synthesis of the corresponding cDNA by means of RT, reagents for the amplification of DNA such as DNA polymerases, dNTPs, buffers, etc.

In another aspect, the invention relates to the use of a kit for diagnosing ascending aorta dilation in a method according to the invention, or for determining the probability that a subject suffers from ascending aorta dilation in a method according to the invention or for determining the prognosis or for monitoring the progression of a subject suffering from ascending aorta dilation in a method according to the invention, wherein the kit comprises reagents adequate for the determination of the level of expression of miR-718 as defined in claim 9.

The invention is detailed below by means of the following examples which are merely illustrative and by no means limiting the scope of the invention.

### EXAMPLES

### Methods

### Study population

The participants included in this study belong to a cohort of patients with BAV prospectively included and followed-up in the inventor's facilities. Upon enrollment, participants were prospectively entered into a specific database, underwent a blood draw and written acceptance. The samples were stored until needed in the inventor's biological samples bank (Biobanc IISPV - HUSJR). BAV diagnosis was made when two aortic leaflets were clearly visualized, with or without a raphe, either on the parasternal short-axis view of a transthoracic echocardiogram, on a transesophageal echocardiogram or on a cardiac magnetic resonance. Explorations were performed or supervised by the same observer.

The design of this study is composed by two evaluations in order to determine the possible miRNAs associated with BAV and aortic dilation. First, the inventors studied the association of BAV on the modulation in the circulating miRNOme profile by the determination, using microarrays, of the miRNA expression profile in plasma of patients diagnosed with BAV (BAV_{non-dil}; n=9), BAV and aortic dilation (BAV_{dil}; n=9), and comparing with the expression profiles of healthy tricuspid aortic valve (TAV) control subjects (TAV_{non-dil}; n=6). For this stage, and in order to improve the study power the inventors selected three groups composed by patients with characteristics very homogenous (Table 2), in terms of excluding possible confounding factors such as age, sex or body mass index (BMI), as well as patients with aortic stenosis, hypertension, diabetes mellitus or pharmacologic treatment (including statins, ACE/ARII and/or β-blockers) were excluded. Also, an aortic diameter lower than 19 mm/m² or higher than 21 mm/m² was used as the criteria to differentiate between the non-dilated or dilated patients respectively.

**Table 2. Clinical and echocardiographic characteristics of the patients included in the microarray.**

| | **Control** | **BAV** | **BAVdil** | **p** |
|---|---|---|---|---|
| Age (years) | 41 ± 4 | 33 ± 3 | 41 ± 4 | 0.177 |
| Sex (male/female) | (6/0) | (6/0) | (6/0) | 1.000 |
| Body weight (kg) | 74.2 ± 2.2 | 75.0 ± 4.2 | 79.0 ± 5.7 | 0.743 |
| Hypertension | 0 (0%) | 0 (0%) | 0 (0%) | 1.000 |
| Hypercholesterolemia | 0 (0%) | 0 (0%) | 2(22.2%) | 0.162 |
| Smoker | 1(20.0%) | 2(22.2%) | 3 (33.3%) | 0.814 |
| Aortic stenosis (mean gradient ≥20 mm | | | | |
| Hg) | 0(0%) | 0(0%) | 0(0%) | 1.00 |
| Aortic regurgitation (≥II) | 0 (0%) | 3 (33.3%) | 6 (66.7%) | 0.031^{∗} |
| Aortic valve gradient (mean, mm Hg) | 3.75 ± 0.3 | 4.6 ± 0.8 | 7.22 ± 1.6 | 0.190 |
| Left ventricle diastolic diameter (mm) | 51.67 ± 1.5 | 50.75 ± 1.0 | 55.11 ± 1.3 | 0.050 |
| Left ventricle systolic diameter (mm) | 32.33 ± 0.9 | 31.88 ± 2.7 | 33.78 ± 1.4 | 0.484 |
| Left ventricular ejection fraction (%) | 74.40 ± 3.2 | 71.25 ± 1.2 | 71.33 ± 2.6 | 0.641 |
| Ascending aorta diameter (mm/m²) | 15.05 ± 0.4 | 15.74 ± 0.7 | 23.35 ± 1.1 | <0.001^{∗∗} |

| | | | | |
|---|---|---|---|---|
| ^{∗} Significant values (p<0.05); ^{∗∗} Significant values (p<0.01); BAV, Bicuspid aortic valve patients; BAVdil, patients with bicuspid aortic valve and aortic dilation. | | | | |

In the second evaluation the inventors validated by RT-qPCR the miRNA candidates in a new cohort of individuals comprised also by TAV (n=19) and BAV individuals (n=24) (Table 3). The BAV group comprised BAV_{non-dil} (n=12) and BAV_{dil} (n=12) patients. The TAV group comprised healthy controls TAV_{non-dil} (n=12) and a new group of patients with TAV and aortic dilation (TAV_{dil}; n=7) in order to determine the specific effects associated with valve morphology or aortic dilation *per se.* Patients with Marfan syndrome were excluded.

**Table 3. Clinical and echocardiographic characteristics of the independent cohort used in the validation study.**

| | **TAV** | **TAVdil** | **BAV** | **BAVdil** | **p** |
|---|---|---|---|---|---|
| Age (years) | 39 ± 3 | 65 ± 3 | 33 ± 3 | 42 ± 3 | <0.001^{∗∗} |
| Sex (male/female) | (8/4) | (5/2) | (11/1) | (6/6) | 0.171 |
| Body weight (kg) | 70.1 ± 3.7 | 76.8 ± 3.4 | 73.25 ± 4.4 | 66.6 ± 4.2 | 0.374 |
| Hypertension | 0 (0%) | 5 (71.4%) | 1(8.3%) | 3 (25%) | 0.002^{∗∗} |
| Hypercholesterolemia | 1(8.3%) | 1(14.3%) | 0 (0%) | 0 (0%) | 0.391 |
| Smoker | 0 (0%) | 0 (0%) | 1(8.3%) | 3 (25%) | 0.105 |
| Aortic stenosis (mean gradient ≥20 mm Hg) | 0 (0%) | 0 (0%) | 1(8.3%) | 2(16.7%) | 0.507 |
| Aortic regurgitation (≥II) | 0(0%) | 4(57.1%) | 7(46.7.3%) | 4(33.3%) | 0.012^{∗} |
| Aortic valve gradient (mean, mm Hg) | 3.4 ± 0.2 | 3.0 ± 0.1 | 6.7 ± 1.0 | 16.5 ± 5.8 | 0.117 |
| Left ventricle diastolic diameter (mm) | 50.1 ± 1.2 | 53.0 ± 2.2 | 54.8 ± 2.0 | 52.3 ± 0.9 | 0.182 |
| Left ventricle systolic diameter (mm) | 29.9 ± 0.7 | 35.8 ± 2.9 | 34.7 ± 0.7 | 32.2 ± 0.7 | 0.048^{∗} |
| Left ventricular ejection fraction (%) | 77.3 ± 1.6 | 61.0 ± 11.7 | 69.1 ± 2.6 | 70.7 ± 1.2 | 0.063 |
| Ascending aorta diameter (mm/m²) | 15.9 ± 0.6 | 23.2 ± 0.9 | 17.6 ± 0.8 | 25.1 ± 1.2 | <0.001^{∗∗} |

| | | | | | |
|---|---|---|---|---|---|
| ^{∗} Significant values (p<0.05); ^{∗∗} Significant values (p<0.01); BAV, Bicuspid aortic valve patients; BAVdil, patients with bicuspid aortic valve and aortic dilation. | | | | | |

### Blood sampling

Blood samples were collected in overnight fasting conditions and were processed within 90 min after. Samples were centrifuged at 1500 g for 15 min to obtain plasma. The plasmas were stored at -80°C until needed in the inventor's biological samples bank (Biobanc IISPV - HUSJR).

### RNA extraction and miRNA microarray preparation

Total RNA was extracted from 250 µl of plasma using the TRIzol reagent according to the manufacturer's instructions (Invitrogen) and purified using RNeasy minikit (Qiagen). To increase RNA recovery 1 µg of MS2 carrier RNA was added to each plasma sample. The quality of total RNA isolated was determined using the Agilent 2100 Bioanalyser.

The plasma miRNA expression levels for 24 individuals were assessed using SurePrint G3 human 8x60k miRNA microarrays (Agilent Techonlogies) covering 1205 human miRNAs (Sanger miRBase release 16). MiRNAs were dephosphorylated and labelled with cyanine 3-cytidine biphosphate including a labelling spike-in solution (Agilent Technologies) to assess the labelling efficiency. The samples were hybridized on the arrays including a hybridization spike-in solution (Agilent Technologies) to monitor hybridization efficiency. Arrays were scanned with a G2565CA Microarray Scanner System with SureScan High Resolution Technology (Agilent Technologies) using Scan Control software. For data processing the Feature extraction 11.5.11 (Agilent Technologies) and GeneSpring 12.6.1 software were used. All steps were performed according to the manufacturer's protocol.

### Microarray analysis of miRNA expression data

The data from microarrays were normalised using the robust multi-array average (RMA) method (Irizarry RA. et al. Biostatistics 2003; 4:249-264), implemented in the AgiMicroRna Bioconductor package, and the fold changes of circulating miRNAs were determined using the linear model implemented in the limma Bioconductor package (Ritchie ME. et al. Nucleic Acids Res 2015; 43:e47). The Benjamini and Hochberg method was used to adjust P-values for multiple testing and control of the false discovery rate (Benjamini Y. and Hochberg Y. J R Stat Soc Ser B 1995; 57:289-300).

### MicroRNA quantification by real-time qRT-PCR

Microarray expression was validated in an independent patient cohort (n=43), including healthy TAV controls, BAV patients, BAVdil patients and an extra group of TAV individuals with aortic dilation. To analyse the expression of each miRNA, reverse transcription was performed using the TaqMan MicroRNA Reverse Transcription Kit (Applied Biosystems) and the miRNA-specific reverse-transcription primers provided with the TaqMan MicroRNA Assay (Applied Biosystems). The final total RNA concentration used was 2.5 ng/µL. The reaction was performed at 16°C for 30 min, 42°C for 30 min and 85°C for 5 min. The inventors used 1.33 µL obtained cDNAs in a subsequent quantitative qRT-PCR amplification using the TaqMan Universal PCR master mix (Applied Biosystems) and the associated specific probe provided in the TaqMan MicroRNA Assay Kit (Applied Biosystems). Specific Taqman probes were used for each gene: microRNA-122-5p (miR-122-5p; hsa-mir-122-5p), 5'-UGGAGUGUGACAAUGGUGUUUG-3' (SEQ ID NO: 1), microRNA-130a-3p (miR-130a-3p; hsa-mir-130a-3p), 5'-CAGUGCAAUGUUAAAAGGGCAU-3' (SEQ ID NO: 2), microRNA-486-5p (miR-486-5p; hsa-mir-486-5p), 5'-UCCUGUACUGAGCUGCCCCGAG-3' (SEQ ID NO: 3), and microRNA-718 (miR-718; hsa-mir-718), 5'-CUUCCGCCCCGCCGGGCGUCG-3' (SEQ ID NO: 4). The results were normalized to the expression of the U6 small nuclear RNA (U6 snRNA), which was used as an endogenous control. Amplification was performed using the ABI Prism 7300 SDS Real-Time PCR system (Applied Biosystems, Madrid, Spain) at 95°C for 10 min, followed by 40 cycles of 95°C for 15 s and 60°C for 1 min. The fold change in the miRNA level was calculated by the log 2 scale according to the equation 2-ΔΔCt, where ΔCt=Ct miRNA-Ct U6 and ΔΔCt=ΔCt treated samples- ΔCt untreated controls.

### Functional and pathways enrichment analysis of miRNA target genes

Target genes of miR-122, miR-130a and miR-486 were predicted using DIANA-miRPath (v3; microrna.gr/tarbase) (Vlachos IS et al. Nucleic Acids Res 2015; 43:W460-466) and, based on an enrichment analysis were mapped on the Kyoto Encyclopaedia of Genes and Genomes (KEGG) pathways and prioritized for the intracellular signal transduction pathways that could be regulated by the three-miRNAs.

Because of the limited target genes identified for miR-718, for this miRNA the inventors combined the target genes predicted by four databases, TargetScan (v7.0; targetscan.org) (Agarwal V et al. Elife 2015; 4:e05005), miRTarBase (2016 version; mirtarbase.mbc.nctu.edu.tw) (Chou C-H et al. Nucleic Acids Res 2015; 44:D239-247), DIANA-microT-CDS (v3; microrna.gr/microT-CDS) (Vlachos IS et al. Nucleic Acids Res 2015; 43:W460-466) and miRDB (2016 version; mirdb.org) (Wong N. and Wang X. Nucleic Acids Res 2015; 43:D146-152) databases. In this case, functional classification of the target genes was carried out with Gene Ontology (GO) and pathway analysis using DAVID (david.ncifcrf.gov) (Huang DW et al. Nat Protoc 2009; 4:44-57) and WebGestalt (webgestalt.org) (Wang J et al. Nucleic Acids Res 2013; 41:W77-83), respectively.

Cytoscape, version 3.0.1 (Shannon P. et al. Genome Res 2003; 13:2498-2504) was used to visualize the predicted miRNA-mRNA interaction. In the networks, pathways of intracellular signal transduction are represented by hexagons-shape nodes, miRNAs are represented as rectangle-shape nodes and target genes are represented as round-shape nodes.

### Statistical analysis

The means of two groups were compared using Student's t test. Chi-squared tests or Fisher exact test, when appropriate, were used to compare the frequencies of the categorical variables. The effects of valve morphology and aortic dilation were assessed using ANOVA. Tukey's test was utilized for pairwise comparisons. Pearson's correlation was used to identify linear relationships between miRNA expression levels and the diameter of the ascending aorta. Backward linear regression models were used to identify independent predictors of the circulating expression of miRNAs. P values <0.05 were considered significant. The statistical analysis was performed using SPSS software, version 21.0 (IBM, Chicago, IL, USA).

### Results

### 1.- Identification of circulating miRNome profiles

Global miRNA expression profiling was performed on plasma samples of TAV controls, BAV_{non-dil} and BAV_{dil} patients in order to identify miRNA candidates, the expressions of which were altered in BAV, because of the valve morphology and/or because of the aortic dilation. The clinical characteristics of patients involved in this stage are provided in Table 1. After processing the microarray data corresponding to the miRNA expression profiles, 260 miRNAs were identified as expressed in at least 10% of the samples. The inventors performed paired comparison between groups to identify miRNA candidates not only for BAV, but also for aortic dilation. In this way, the inventors firstly analysed the effect of the valve morphology (comparing patients with TAV_{non-dil} and BAV_{non-dil}) on the miRNome, and as it is reported in the volcano plot (Figure 1A), it was found that the expression of miR-122-5p and miR-486-5p significantly differ from the rest, indicating that the expression levels of this two miRNAs in plasma could be associated with the morphology of the aortic valve.

In a second analysis the inventors compared the two most divergent groups (TAV_{non-dil} and BAV_{dil}) in order to integrate the complexity associated to the valve morphology and the dilation of the ascending aorta. Using this analysis it was found that the expression of other two miRNAs, the miR-130a-3p and the miR-718 (Figure 1B), were significantly different between the plasmas of TAV_{non-dil} and BAV_{dil} patients.

### 2.- Validation by RT-qPCR of miRNAs candidates in an independent cohort

The miRNA candidates selected using the microarray approach were validated by RT-qPCR in a new set (n=43) comprised by TAV_{non-dil} healthy controls, BAV_{non-dil}, BAV_{dil} patients and, in order to determine the specific pattern associated with the valve morphology or aortic dilation *per se,* a new group of patients with TAV and aortic dilation (TAV_{dil}) was included.

### 2.1- The expression levels of miR-122-5p, miR-130a-3p and miR-486-5p are associated with the morphology of the aortic valve

On one hand, the inventors compared the expression in plasma of the miRNA candidates, miR-122-5p, miR-130a-3p, miR-486-5p and miR-718, depending on the morphology of the valve with independence on the presence or absence of dilation in the ascending aorta (Figure 2A).

Using this validation study the inventors corroborated that the expression of three of the miRNA candidates, miR-122-5p, miR-130a-3p, miR-486-5p, differ significantly between the plasma of TAV healthy controls and BAV patients (BAV vs. TAV), therefore their circulating expression levels were influenced by the morphology of the valve. The inventors observed that bicuspid valve morphology was associated with decreased circulating levels of miR-122-5p, miR-486-5p, instead the increased levels reported for miR-130a-3p.

In order to deep insight into the biological role of miR-122, miR-130a and miR-486 in BAV, a functional categorization of miRNAs target genes was performed. Targets of each selected miRNA were predicted and based on an enrichment analysis were mapped on KEGG pathways and prioritized the intracellular signal transduction pathways that could be regulated by the three-miRNA. Using this approach the inventors identified the transforming growth factor-beta (TGF-β) signalling pathway as the most significantly enriched (p=7.73 × 10⁻⁷) with a total of 32 genes targeted by the four selected miRNAs (Figure 2B).

### 2.2.- Role of aortic dilation on the miR-130a-3p and miR-718 expression levels in plasma

On the other hand, the inventors validated whether the modulation of the miRNA expression profile of miR-130a-3p and miR-718 in plasma would be related to the dilation of the aorta. The inventors firstly analysed the effect of the aortic dilation on the miR-130a-3p and miR-718 comparing the expression levels between dilated and non-dilated patients independently of the aortic valve morphology (n=43, Figure 3A), and they observed that the up-regulation of miR-130a-3p expression in plasma of BAV patients was only influenced by the morphology of the aortic valve and that the dilation of the aorta had no modulatory effect. In contrast, the inventors observed that miR-718 plasma expression was significantly affected by the dilation of the ascending aorta, and that this dilation-promoted effect was hiding the differential expression consequence of the valve morphology not observed in the comparisons used in previous validation.

The inventors explored these morphology- and aortic dilation-mediated effects by comparing the expression of miR-130a-3p (Figure 3B) and miR-718 (Figure 3C) between the four sub-groups depending on the valve morphology and the dilation of the ascending aorta (TAV_{non-dil}, TAV_{dil}, BAV_{non-dil} and BAV_{dil}). Using this analysis the inventors corroborated that up-regulated miR-130a-3p expression was associated only to a bicuspid morphology of the aortic valve, and no effect of the aortic dilation was observed. The inventors also corroborated that the down-regulated expression of miR-718 observed in BAV patients was significantly more accused in presence of aortic dilation in comparison with non-aortic dilated ones, with independence of the tricuspid or bicuspid aortic valve morphology.

### 3.- The expression of miR-718 in plasma is α predictor of aortic dilation

The role of miR-718 as biomarker of aortic dilation was supported through the determination that the miR-718 plasma expression was inversely correlated with the diameter of the ascending aorta (R=-0.629, p=3.1×10⁻⁵ and Figure 4), using all patients included in the validation cohort (n=43), independently of their valve morphology (TAV and BAV) or the presence or absence of aortic dilation (dilated and non-dilated). Furthermore, using this validation cohort, the inventors built a multivariate linear model using the miR-718 expression profile as predictive variables and the dilation of the aorta (represented as categorical variable or as the diameter of the ascending aorta) as outcome variable as well as the age and hypertension as possible confounding factors (Table 4). Using this analysis the inventors confirmed that the miR-718 expression in plasma is an independent predictor of the dilation of the aorta.

**Table 4. A summary of the results of the multivariate linear regression analysis of miR-718 expression levels as predictor of aortic dilation.**

| | Aortic dilation | |
|---|---|---|
| | β | p |
| Age (years) | 0.317 | 0.118 |
| Hypertension | 0.050 | 0.801 |
| miR-718 expression | -0.407 | 0.022^{∗} |

The analysis was performed in the independent validation cohort, including TAV, TAVdil, BAV and BAVdil (n=43).^{∗} Significant values (p<0.05)

In order to provide an improved understanding of the possible functional relevance of miR-718 down-regulation, the inventors characterized the putative biological functions of this miRNA based on the identification of miR-718 target genes. The predicted miRNA-gene interaction network is composed by the miR-718 and by 167 target genes (data not shown). The inventors also performed a functional enrichment analysis to predict the potential biological function of the miR-718 target genes. DAVID was used to determine gene ontologies (GO) associated to biological processes that would be overrepresented within the miR-718 target genes and the inventors found that four of the target genes were significantly enriched for the GO "blood vessel remodelling" biological process (p=1.9×10⁻²). Using WebGestalt the inventors performed a functional analysis based on the pathways that could be associated with miR-718 targets and they identified that 3 of the miR-718 target genes were significantly enriched for the "focal adhesion pathway" (p=9.0×10⁻⁴). Vascular endothelial growth factor A (VEGFA) is one of the miR-718 target genes that are significantly enriched for the "focal adhesion pathway".

## Claims

1. An *in vitro* method for diagnosing ascending aorta dilation in a subject that comprises
(i) determining the level of expression of circulating miR-718 in a blood sample from said subject, and
(ii) comparing the level of expression obtained in (i) with a reference value, wherein if the level of expression of circulating miR-718 is decreased with respect to the reference value, then the subject is diagnosed with ascending aorta dilation.

2. The method according to claim 1, wherein the reference value is the level of expression of miR-718 determined in a blood sample from one or more subjects suffering from a non-dilated bicuspid aortic valve or in a blood sample from one or more non-dilated tricuspid aortic valve healthy subjects.

3. The method according to any one of claims 1 or 2, wherein the subject diagnosed with ascending aorta dilation is selected from the group consisting of a subject having a bicuspid aortic valve and a subject having a tricuspid aortic valve.

4. An *in vitro* method for determining the probability that a subject suffers from ascending aorta dilation that comprises
(i) determining the level of expression of circulating miR-718 in a blood sample from said subject, and
(ii) comparing the level of expression obtained in (i) with a reference value, wherein if the level of expression of circulating miR-718 is decreased with respect to the reference value, then the subject shows high probability of suffering from ascending aorta dilation or
wherein if the level of expression of circulating miR-718 is equal to or increased with respect to the reference value, then the subject shows low probability of suffering from ascending aorta dilation.

5. The method according to claim 4, wherein the reference value is the level of expression of miR-718 determined in a blood sample from one or more subjects having an ascending aortic diameter lower than 19 mm/m².

6. An *in vitro* method for determining the prognosis or for monitoring the progression of a subject suffering from ascending aorta dilation that comprises:
(i) determining the level of expression of circulating miR-718 in a blood sample from said subject, and
(ii) comparing the level of expression obtained in (i) with a reference value obtained from the same subject at an earlier time of point
wherein if the level of expression of circulating miR-718 is decreased with respect to the reference value, then the subject shows bad prognosis or
wherein if the level of expression of circulating miR-718 is increased with respect to the reference value, then the subject shows good prognosis.

7. The method according to any one of claims 1 to 6, wherein the blood sample is a plasma sample.

8. The method according to any one of claims 1 to 7, wherein the subject is a human.

9. A kit for use in the method of any of claims 1-8
comprising reagents adequate for the determination of the level of expression of miR-718, wherein the oligonucleotides that hybridize specifically to miR-718 comprise at least 50% of the total amount of oligonucleotides forming the kit.

10. The kit according to claim 9, further comprising reagents adequate for the determination of the level of expression of one or more reference RNAs and/or reagents adequate for the determination of the level of expression of one or more constitutive genes.

11. Use of a kit for diagnosing ascending aorta dilation in a method according to any one of claims 1 to 3, or for determining the probability that a subject suffers from ascending aorta dilation in a method according to any one of claims 4 or 5, or for determining the prognosis or for monitoring the progression of a subject suffering from ascending aorta dilation in a method according to claim 6, wherein the kit comprises reagents adequate for the determination of the level of expression of miR-718.

12. Use according to claim 11, wherein the reagents adequate for the determination of the level of expression of miR-718 are oligonucleotides that hybridize specifically to said miR-718.

13. Use according to any one of claims 11 or 12, wherein the kit further comprises reagents adequate for the determination of the level of expression of one or more reference RNAs and/or reagents adequate for the determination of the level of expression of one or more constitutive genes.

## Patentansprüche

1. Ein in-vitro-Verfahren zur Diagnose der Dilatation der aufsteigenden Aorta bei einem Subjekt, das umfasst
(i) Bestimmen des Expressionsniveaus von zirkulierendem miR-718 in einer Blutprobe des Subjekts, und
(ii) Vergleichen des in (i) erhaltenen Expressionsniveaus mit einem Referenzwert, wobei, wenn das Expressionsniveau von zirkulierendem miR-718 in Bezug auf den Referenzwert vermindert ist, bei dem Subjekt eine Dilatation der aufsteigenden Aorta diagnostiziert wird.

2. Das Verfahren nach Anspruch 1, wobei der Referenzwert das Expressionsniveau von miR-718 ist, das in einer Blutprobe von einem oder mehreren Subjekten, die an einer nicht dilatierten bikuspiden Aortenklappe leiden, oder in einer Blutprobe von einem oder mehreren gesunden Subjekten mit nicht dilatierter trikuspider Aortenklappe bestimmt wurde.

3. Das Verfahren nach einem der Ansprüche 1 oder 2, wobei das Subjekt, bei dem eine Dilatation der aufsteigenden Aorta diagnostiziert wird, aus der Gruppe ausgewählt wird, die aus einem Subjekt mit einer bikuspiden Aortenklappe und ein Subjekt mit einer trikuspiden Aortenklappe besteht.

4. Ein in-vitro-Verfahren zur Bestimmung der Wahrscheinlichkeit, dass ein Subjekt an einer Dilatation der aufsteigenden Aorta leidet, das umfasst
(i) Bestimmen des Expressionsniveaus von zirkulierendem miR-718 in einer Blutprobe des Subjekts, und
(ii) Vergleichen des in (i) erhaltenen Expressionsniveaus mit einem Referenzwert,
wobei, wenn das Expressionsniveau von zirkulierendem miR-718 in Bezug auf den Referenzwert vermindert ist, das Subjekt eine hohe Wahrscheinlichkeit aufweist, an einer Dilatation der aufsteigenden Aorta zu leiden, oder
wobei, wenn das Expressionsniveau von zirkulierendem miR-718 in Bezug auf den Referenzwert gleich oder erhöht ist, das Subjekt eine geringe Wahrscheinlichkeit aufweist, an einer Dilatation der aufsteigenden Aorta zu leiden.

5. Das Verfahren nach Anspruch 4, wobei der Referenzwert das Expressionsniveau von miR-718 ist, das in einer Blutprobe von einem oder mehreren Subjekten mit einem Durchmesser der aufsteigenden Aorta von weniger als 19 mm/m² bestimmt wurde.

6. Ein in-vitro-Verfahren zur Bestimmung der Prognose oder zur Überwachung des Verlaufs eines Subjekts, das an einer Dilatation der aufsteigenden Aorta leidet, das umfasst:
(i) Bestimmen des Expressionsniveaus von zirkulierendem miR-718 in einer Blutprobe des Subjekts, und
(ii) Vergleichen des in (i) erhaltenen Expressionsniveaus mit einem Referenzwert, der von demselben Subjekt zu einem früheren Zeitpunkt erhalten wurde,
wobei, wenn das Expressionsniveau von zirkulierendem miR-718 in Bezug auf den Referenzwert verringert ist, das Subjekt eine schlechte Prognose aufweist, oder
wobei, wenn das Expressionsniveau von zirkulierendem miR-718 in Bezug auf den Referenzwert erhöht ist, das Subjekt eine gute Prognose aufweist.

7. Das Verfahren nach einem der Ansprüche 1 bis 6, wobei die Blutprobe eine Plasmaprobe ist.

8. Das Verfahren nach einem der Ansprüche 1 bis 7, wobei das Subjekt ein Mensch ist.

9. Ein Kit zur Verwendung in dem Verfahren nach einem der Ansprüche 1-8, umfassend Reagenzien, die für die Bestimmung des Expressionsniveaus von miR-718 geeignet sind, wobei die Oligonukleotide, die spezifisch an miR-718 hybridisieren, mindestens 50% der Gesamtmenge der das Kit bildenden Oligonukleotide ausmachen.

10. Das Kit nach Anspruch 9, das ferner umfasst Reagenzien, die für die Bestimmung des Expressionsniveaus einer oder mehrerer Referenz-RNAs geeignet sind, und/oder Reagenzien, die für die Bestimmung des Expressionsniveaus eines oder mehrerer konstitutiver Gene geeignet sind.

11. Verwendung eines Kits zur Diagnose der Dilatation der aufsteigenden Aorta in einem Verfahren nach einem der Ansprüche 1 bis 3 oder zur Bestimmung der Wahrscheinlichkeit, dass ein Subjekt an einer Dilatation der aufsteigenden Aorta leidet, in einem Verfahren nach einem der Ansprüche 4 oder 5 oder zur Bestimmung der Prognose oder zur Überwachung des Verlaufs eines Subjekts, das an einer Dilatation der aufsteigenden Aorta leidet, in einem Verfahren nach Anspruch 6, wobei das Kit Reagenzien umfasst, die für die Bestimmung des Expressionsniveaus von miR-718 geeignet sind.

12. Verwendung nach Anspruch 11, wobei die Reagenzien, die für die Bestimmung des Expressionsniveaus von miR-718 geeignet sind, Oligonukleotide sind, die spezifisch an das miR-718 hybridisieren.

13. Verwendung nach einem der Ansprüche 11 oder 12, wobei das Kit ferner Reagenzien umfasst, die für die Bestimmung des Expressionsniveaus einer oder mehrerer Referenz-RNAs und/oder Reagenzien, die für die Bestimmung des Expressionsniveaus eines oder mehrerer konstitutiver Gene geeignet sind.

## Revendications

1. Un procédé *in vitro* pour diagnostiquer une dilatation de l'aorte ascendante chez un sujet, qui comprend
(i) le fait de déterminer le niveau d'expression de miR-718 circulant dans un échantillon de sang provenant dudit sujet, et
(ii) le fait de comparer le niveau d'expression obtenu en (i) avec une valeur de référence ;
si le niveau d'expression de miR-718 circulant est diminué par rapport à la valeur de référence, alors le sujet est diagnostiqué comme ayant une dilatation de l'aorte ascendante.

2. Le procédé selon la revendication 1, dans lequel la valeur de référence est le niveau d'expression de miR-718 déterminé dans un échantillon de sang provenant d'un ou plusieurs sujets souffrant d'une valve bicuspide aortique non dilatée ou dans un échantillon de sang provenant d'un ou plusieurs sujets sains à valve aortique tricuspide non dilatée.

3. Le procédé selon l'une quelconque des revendications 1 ou 2, dans lequel le sujet diagnostiqué avec une dilatation de l'aorte ascendante est choisi dans le groupe consistant en un sujet ayant une valve aortique bicuspide et un sujet ayant une valve aortique tricuspide.

4. Un procédé *in vitro* pour déterminer la probabilité qu'un sujet souffre d'une dilatation de l'aorte ascendante, qui comprend
(i) le fait de déterminer le niveau d'expression de miR-718 circulant dans un échantillon de sang provenant dudit sujet, et
(ii) le fait de comparer le niveau d'expression obtenu en (i) avec une valeur de référence ;
si le niveau d'expression de miR-718 circulant est diminué par rapport à la valeur de référence, alors le sujet présente une probabilité élevée de souffrir d'une dilatation de l'aorte ascendante ou
si le niveau d'expression du miR-718 circulant est égal ou augmenté par rapport à la valeur de référence, alors le sujet présente une faible probabilité de souffrir d'une dilatation de l'aorte ascendante.

5. Le procédé selon la revendication 4, dans lequel la valeur de référence est le niveau d'expression de miR-718 déterminé dans un échantillon sanguin provenant d'un ou plusieurs sujets ayant un diamètre aortique ascendant inférieur à 19 mm/m².

6. Un procédé *in vitro* pour déterminer le pronostic ou pour suivre la progression d'un sujet souffrant d'une dilatation de l'aorte ascendante, qui comprend :
(i) le fait de déterminer le niveau d'expression de miR-718 circulant dans un échantillon de sang provenant dudit sujet, et
(ii) le fait de comparer le niveau d'expression obtenu en (i) avec une valeur de référence obtenue à partir du même sujet à un moment antérieur ;
si le niveau d'expression du miR-718 circulant est diminué par rapport à la valeur de référence, alors le sujet présente un mauvais pronostic ou
si le niveau d'expression de miR-718 circulant est augmenté par rapport à la valeur de référence, alors le sujet présente un bon pronostic.

7. Le procédé selon l'une quelconque des revendications 1 à 6, dans lequel l'échantillon de sang est un échantillon de plasma.

8. Le procédé selon l'une quelconque des revendications 1 à 7, dans lequel le sujet est un être humain.

9. Un kit destiné à être utilisé dans le procédé selon l'une quelconque des revendications 1 à 8,
comprenant des réactifs adéquats pour la détermination du niveau d'expression de miR-718, les oligonucléotides qui s'hybrident spécifiquement à miR-718 comprenant au moins 50 % de la quantité totale d'oligonucléotides formant le kit.

10. Le kit selon la revendication 9, comprenant en outre des réactifs adéquats pour la détermination du niveau d'expression d'un ou plusieurs ARN de référence et/ou des réactifs adéquats pour la détermination du niveau d'expression d'un ou plusieurs gènes constitutifs.

11. Utilisation d'un kit pour diagnostiquer une dilatation de l'aorte ascendante dans un procédé selon l'une quelconque des revendications 1 à 3, ou pour déterminer la probabilité qu'un sujet souffre de dilatation de l'aorte ascendante dans un procédé selon l'une quelconque des revendications 4 ou 5, ou pour déterminer le pronostic ou pour surveiller la progression d'un sujet souffrant d'une dilatation de l'aorte ascendante dans un procédé selon la revendication 6, dans lequel le kit comprend des réactifs adéquats pour la détermination du niveau d'expression de miR-718.

12. Utilisation selon la revendication 11, dans laquelle les réactifs adéquats pour la détermination du niveau d'expression de miR-718 sont des oligonucléotides qui s'hybrident spécifiquement audit miR-718.

13. Utilisation selon l'une quelconque des revendications 11 ou 12, dans laquelle le kit comprend en outre des réactifs adéquats pour la détermination du niveau d'expression d'un ou plusieurs ARN de référence et/ou des réactifs adéquats pour la détermination du niveau d'expression d'un ou plusieurs gènes constitutifs.


SEQUENCE LISTING

<110>  Fundació Institut d'Investigació Sanitària Pere Virgili

<120>  PREDICTIVE METHODS OF ASCENDING AORTA DILATION AND BICUSPID
       AORTIC VALVE

<130>  P13815PC00

<150> EP 17382048.1
<151> 2017-02-06

<160>  4

<170>  PatentIn version 3.5

<210>  1
<211>  22
<212>  RNA
<213>  Artificial Sequence

<220>
<223>  Taqman probe for microRNA-122-5p

<400>  1
uggaguguga caaugguguu ug                                              22


<210>  2
<211>  22
<212>  RNA
<213>  Artificial Sequence

<220>
<223>  Taqman probe for microRNA-130a-3p

<400>  2
cagugcaaug uuaaaagggc au                                              22


<210>  3
<211>  22
<212>  RNA
<213>  Artificial Sequence

<220>
<223>  Taqman probe for microRNA-486-5p

<400>  3
uccuguacug agcugccccg ag                                              22


<210>  4
<211>  21
<212>  RNA
<213>  Artificial Sequence

<220>
<223>  Taqman probe for microRNA-718

<400>  4
cuuccgcccc gccgggcguc g                                               21